# EUROPEAN PATENT APPLICATION

(11) **EP 1 704 858 A1**
(43) Date of publication of application: **27.09.2006**
(21) Application number: 05703332.6
(22) Date of filing: 06.01.2005
(51) Int. Cl.: A61K 31/05, A61K 9/10, A61K 47/24, A61K 47/40, A61K 47/44, A61P 25/20

(54) **PROPOFOL-CONTAINING FAT EMULSION PREPARATION**

(30) Priority: 14.01.2004 JP 2004006409
(71) Applicant: OTSUKA PHARMACEUTICAL FACTORY, INC., Naruto-shi, Tokushima 772-8601 (JP)
(72) Inventor: TAKEDA, Koichi, Itano-gun, Tokushima 7710204 (JP); MATSUDA, Kenji Green Shato Rapyuta 105 60-2, Aza, Itano-gun, Tokushima 7710218 (JP); TERAO, Toshimitsu, Naruto-shi, Tokushima 7720015 (JP); INOUE, Tadaaki, Tokushima-shi, Tokushima 7700021 (JP); IMAGAWA, Takashi, Hyogo 6511123 (JP); MASUMI, Shigeru, Naruto-shi, Tokushima 7720017 (JP)
(74) Representative: HOFFMANN EITLE
(86) International application number: PCT/JP2005/000064
(87) International publication number: WO 2005/067905

(57) **Abstract**

The present invention provides a propofol-containing fat emulsion that can be administered with reduced vascular pain without incorporating a local anesthetic such as lidocaine; and a process for producing the same. The fat emulsion comprises 0.1 to 5 w/v% of propofol, 2 to 20 w/v% of an oily component, 0.4 to 10 w/v% of an emulsifier and 0.02 to 0.3 w/v% of at least one compound selected from the group consisting of cyclodextrins, cyclodextrin derivatives and pharmacologically acceptable salts thereof, and is in the form of a fat emulsion.

## Description

### TECHNICAL FIELD

The present invention relates to a propofol-containing fat emulsion that can be administered with reduced vascular pain.

### BACKGROUND ART

Propofol (2,6-diisopropylphenol) is a lipophilic substance that induces hypnosis. Because of its poor solubility in water, propofol is usually prepared in the form of an oil-in-water fat emulsion using an oily component and an emulsifier, the emulsion being capable of being administered directly into the bloodstream either by intravenous injection or infusion. Such a propofol-containing fat emulsion is widely used as a general anesthetic, sedative, and the like (see, for example, U.S. Patent No. 5,714,520). Known commercially available products thereof include, for example, 1% Diprivan™ injection (AstraZeneca).

However, it has been reported that a strong sharp pain (vascular pain) frequently develops as a side effect during the administration of such preparations by intravenous injection or infusion (see, for example, W. Klemment, J.O. Arndt, British Journal of Anaesthesia, 1991, 67: 281-284).

This problem can be solved by incorporating into the fat emulsion a local anaesthetic, such as lidocaine, in an amount effective to remove pain. However, conventional propofol-containing fat emulsions have the drawback that when a local anaesthetic such as lidocaine is mixed therewith, the stability of the emulsion is rapidly lost and emulsion particles agglomerate in a short time, usually within 30 minutes, and the emulsion breaks down and separates into water and oil phases, so that the emulsion cannot be administered by injection or infusion (see E.E.M. Lilley et al., Anaesthesia, 1996, 51: 815-818).

To overcome this serious drawback, i..e., sharply reduced emulsion stability caused by the incorporation of lidocaine, etc., a technique has been proposed in which a hydrophilic surfactant having an HLB value of 10 or more, such as polyoxyethylene (60) hydrogenated castor oil, etc., is incorporated as a stabilizer into the aqueous phase of a propofol-containing fat emulsion, and the pH is adjusted to within the range of 3.0 to 6.5 (see Japanese Unexamined Patent Publication No. 2002-179562). The proposed technique is capable of preventing emulsion breakdown in the obtained fat emulsion to some extent but has the disadvantage that the obtained fat emulsion has a safety problem because the hydrophilic surfactant used as a stabilizer has low safety.

Aqueous solutions comprising propofol and a specific cyclodextrin compound, and lyophilized products thereof have been proposed as propofol-containing preparations that can be administered with reduced vascular pain (see, for example, European Patent Publication No. 02/074200 (U.S. Patent Application Publication No. 2003-73665), and European Patent Publication No. 03/063824).

However, these proposed preparations are in the form of aqueous solutions (or lyophilized products thereof) that do not contain an oily component. These disclosed techniques are intended to prevent or alleviate the occurrence of vascular pain by making a preparation in the form of an aqueous solution, not in the form of a fat emulsion, and therefore are not intended to prevent or alleviate the occurrence of vascular pain during the administration of a propofol-containing fat emulsion without impairing the stability of the emulsion (emulsion stability). Furthermore, since propofol is a lipophilic substance and has the characteristic of being poorly soluble in water, the cyclodextrin compound needs to be used in a large amount, at least equimolar to propofol, and about 3 to 80 w/v% of the entire aqueous solution. However, when using preparations containing such a large amount of cyclodextrin compound, toxicity and side effects of the cyclodextrin compound cannot be ignored. In fact, it is known that the administration of such a cyclodextrin-containing preparation causes problems such as osmotic pressure elevation and increased hemolysis (see, for example, the above-mentioned U.S. Patent Application Publication No. 2003-73665).

Thus, a propofol-containing fat emulsion that prevents or alleviates the occurrence of sharp pain, etc. during administration without impairing emulsion stability and safety has not yet been developed.
Patent Document 1: Japanese Unexamined Patent Publication No. 2002-179562
Patent Document 2: U.S. Patent Application Publication No. 2003-73665
Patent Document 3: European Patent Publication No. 03/063824 Non-patent document 1: W. Klemment, J. O. Arndt, British Journal of Anaesthesia, 1991, 67: 281-284
Non-patent document 2: E. E. M. Lilley, et al., Anaesthesia, 1996, 51: 815-818

### DISCLOSURE OF THE INVENTION

### PROBLEM TO BE SOLVED BY THE INVENTION

An object of the present invention is to provide an improved propofol-containing fat emulsion with high safety and excellent emulsion stability, the emulsion being administered with reduced vascular pain.

### MEANS FOR SOLVING THE PROBLEM

The present inventors carried out extensive research and found that a propofol-containing fat emulsion prepared by mixing propofol, an oily component, an emulsifier and a cyclodextrin compound in specific amounts can achieve the above object. The invention has been accomplished based on this finding and further research. The present invention provides the following fat emulsions and processes of preparing the fat emulsions according to items 1 to 11.

Item 1. A propofol-containing fat emulsion capable of being administered with reduced vascular pain during intravenous or infusion administration, the emulsion comprising 0.1 to 5 w/v% of propofol, 2 to 20 w/v% of an oily component, 0.4 to 10 w/v% of an emulsifier, and 0.02 to 1 w/v% of at least one compound (hereinafter sometimes simply referred to as "cyclodextrin compound") selected from the group consisting of cyclodextrins, cyclodextrin derivatives and pharmacologically acceptable salts thereof.

Item 2. The fat emulsion according to item 1 wherein the oily component is at least one compound selected from the group consisting of natural triglycerides and synthetic triglycerides, and the emulsifier is at least one member selected from the group consisting of natural phospholipids and synthetic phospholipids.

Item 3. The fat emulsion according to item 2 wherein the oily component is soybean oil, and the emulsifier is egg yolk lecithin.

Item 4. The fat emulsion according to item 1 wherein the cyclodextrin compound is at least one member selected from the group consisting of 2-hydroxypropyl-β-cyclodextrin and sulfobutylether-β-cyclodextrin.

Item 5. The fat emulsion according to item 1 wherein the oily component is soybean oil, the emulsifier is egg yolk lecithin, and the cyclodextrin compound is at least one member selected from the group consisting of 2-hydroxypropyl-β-cyclodextrin and sulfobutylether-β-cyclodextrin.

Item 6. The fat emulsion according to item 1 comprising 0.5 to 3 w/v% of propofol, 3 to 10 w/v% of the oily component, 0.5 to 7 w/v% of the emulsifier, and 0.05 to 0.5 w/v% of the cyclodextrin compound.

Item 7. The fat emulsion according to item 1 comprising 0.5 to 3 w/v% of propofol, 3 to 10 w/v% of the oily component, 0.5 to 7 w/v% of the emulsifier, and 0.05 to 0.2 w/v% of the cyclodextrin compound.

Item 8. A process of preparing the fat emulsion of item 1 comprising emulsifying a mixture comprising propofol, the oily component and the emulsifier in water, and then adding the cyclodextrin compound to the obtained emulsion.

Item 9. A process of preparing the fat emulsion of item 1 comprising adding a mixture comprising propofol, the oily component and the emulsifier to an aqueous solution of the cyclodextrin compound, and then emulsifying the obtained mixture.

Item 10. A method of reducing vascular pain caused by intravenous or infusion administration of a fat emulsion comprising 0.1 to 5 w/v% of propofol, 2 to 20 w/v% of an oily component, and 0.4 to 10 w/v% of an emulsifier, the method comprising incorporating 0.02 to 1 w/v% of a cyclodextrin compound into the fat emulsion.

Item 11. Use of a cyclodextrin compound for preparing a propofol-containing fat emulsion capable of being administered with reduced vascular pain during intravenous or infusion administration, the emulsion comprising 0.1 to 5 w/v% of propofol, 2 to 20 w/v% of an oily component, 0.4 to 10 w/v% of an emulsifier, and 0.02 to 1 w/v% of the cyclodextrin compound.

The prevent invention has been accomplished based on the following findings: when using an oily component, an emulsifier and a cyclodextrin compound in specific proportions, interaction of these components produces a fat emulsion with excellent emulsion stability, and furthermore, the obtained emulsion does not have a serious defect, i.e., occurrence of vascular pain as a side effect during administration, which has unavoidably occurred in conventional propofol-containing fat emulsions.

More specifically, the fat emulsion of the present invention has the following advantages:
(1) remarkably reducing side effects such as vascular pain during administration without incorporating a local anesthetic such as lidocaine;
(2) preventing emulsion stability reduction caused by incorporation of lidocaine or the like; and
(3) being highly safe because it is unnecessary to incorporate low-safety surfactants such as polyoxyethylene (60) hydrogenated castor oil to improve the emulsion stability reduction.

Details of these specific effects of the fat emulsion of the invention are as described in the Examples below.

The propofol-containing fat emulsion of the invention is described below in more detail. Propofol

Propofol is a compound known in the field of medicine as a general anesthetic or sedative, as described in the above Japanese Unexamined Patent Publication No. 2002-179562. The solubility of this compound in water is quite low in view of the effective amount to be administered. In the fat emulsion of the invention, the propofol is usually contained in an amount of 0.1 to 5 w/v%.

In this specification, "w/v%" used to describe the amount of each component of the fat emulsion of the invention refers to the weight of the component in grams per 100 ml volume of the entire fat emulsion.

### Oily component and emulsifier

In the fat emulsion of the invention, vegetable oils (natural triglycerides) can be used as the oily component (fat). Specific examples of such vegetable oils include soybean oil, cotton seed oil, rapeseed oil, sesame oil, safflower oil, corn oil, peanut oil, olive oil, coconut oil, perilla oil, castor oil, and the like.

Synthetic triglycerides such as 2-linoleoyl-1,3-dioctanoyl glycerol, and medium chain triglycerides (MCTs) such as C₈₋₁₀ triglycerides are also usable as the oily component. Examples of commercially available products mainly containing such medium chain triglycerides (MCTs) include "COCONARD™" (trade name, product of Kao Corporation), "ODO™" (trade name, product of Nisshin Oil Mills, Ltd.), "Miglyol™" (trade name, product of SASOL Ltd.), "Panasate™" (trade name, product of NOF Corporation), and the like.

The oily component is not limited to the above-mentioned vegetable oils and medium chain triglycerides, and other examples of usable oily components include animal oils, mineral oils, synthetic oils, and essential oils.

Such oily components can be used singly or as a combination of two or more. When such oily components are used in combination, they do not have to be selected from the same group such as vegetable oils, medium chain triglycerides, animal oils, mineral oils, or the like, but can be selected from different groups.

Typical examples of emulsifiers include natural phospholipids such as egg yolk lecithin, egg yolk phosphatidylcholine, soybean lecithin and soybean phosphatidylcholine, and hydrogenated products thereof such as hydrogenated egg yolk lecithin, hydrogenated egg yolk phosphatidylcholine, hydrogenated soybean lecithin and hydrogenated soybean phosphatidylcholine. Synthetic phospholipids may also be used as the emulsifier. Examples of synthetic phospholipids include phosphatidylcholines (e.g., dipalmitoylphosphatidylcholine, dimyristoylphosphatidylcholine, distearoylphosphatidylcholine, dioleoylphosphatidylcholine), phosphatidylglycerols (e.g., dipalmitoylphosphatidylglycerol, dimyristoylphosphatidylglycerol, distearoylphosphatidylglycerol, dioleoylphosphatidylglycerol), phosphatidylethanolamines (e.g., dipalmitoylphosphatidylethanolamine, dimyristoylphosphatidylethanolamine, distearoylphosphatidylethanolamine, dioleoylphosphatidylethanolamine), and the like.

Such emulsifiers can be used singly or as a combination of two or more. Among these, preferable are egg yolk lecithin, egg yolk phosphatidylcholine, soybean lecithin and soybean phosphatidylcholine.

The oily component and the emulsifier may be in the form of an emulsion which has been prepared by mixing them and emulsifying the mixture in water. Methods for preparing such an emulsion (emulsification methods) are well known in this field. Examples of usable methods include a process comprising the steps of adding water for injection to a mixture of the oily component and the emulsifier to form a crude emulsion, and emulsifying (refining) the crude emulsion using a suitable high-pressure emulsifying device, etc. More specifically, the crude emulsion can be obtained by stirring the mixture at 5000 rpm or more for at least 5 minutes using, for example, a homomixer such as a T.K. homomixer manufactured by Tokushu Kika Kogyo Co., Ltd. The crude emulsion can be refined by using a high-pressure homogenizer, an ultrasonic homogenizer, or the like. When using a high-pressure homogenizer, the crude emulsion can be usually refined by passing through the homogenizer about 2 to 50 times, and preferably about 5 to 20 times, at a pressure of about 200 kg/cm² or more. The mixing and emulsifying procedures can be carried out at room temperature or with slight heating (usually about 40°C to 80°C).

The proportions of oily component and emulsifier are not particularly limited as long as an emulsion can be obtained. The oily component is usually contained in the fat emulsion of the invention in the range of 2 to 20 w/v%, and preferably 3 to 10 w/v%, and the emulsifier in the range of 0.4 to 10 w/v%, and preferably 0.5 to 7 w/v%. The desired stable propofol-containing fat emulsion of the invention that prevents or alleviates vascular pain can be obtained by using these components in the above-mentioned ranges.

### Cyclodextrin compound

It is important that the fat emulsion of the invention contains at least one compound selected from the group consisting of cyclodextrins, cyclodextrin derivatives, and pharmacologically acceptable salts thereof. Cyclodextrins as used herein refer to cyclic oligosaccharides composed of 6 to 12 glucose units. Cyclodextrin derivatives refer to compounds in which a portion or all of the hydroxy groups at the 2-, 3-, and 6-positions of the glucose units constituting the cyclodextrin are substituted by other functional groups. Specific examples of cyclodextrins and cyclodextrin derivatives include compounds represented by the following formula (1): 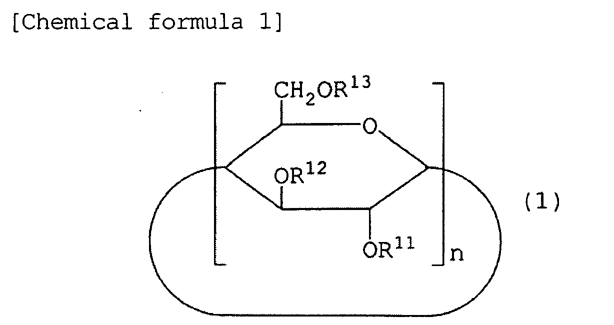 wherein n is an integer from 6 to 12; R¹¹, R¹² and R¹³
are the same or different and represent hydrogen, alkyl, monohydroxyalkyl, dihydroxyalkyl, sulfoalkyl, carboxyalkyl or a sugar moiety, n R¹¹s may be the same or different, n R¹²s may be the same or different, and n R¹³s may be the same or different.

Examples of alkyl groups include C₁₋₄ alkyl groups such as methyl, ethyl, propyl, and the like. Examples of monohydroxylalkyl groups include monohydroxy-C₁₋₄ alkyl groups such as hydroxymethyl, 2-hydroxyethyl, 2-hydroxypropyl and the like. Examples of dihydroxyalkyl groups include dihydroxy-C₁₋₄ alkyl groups such as dihydroxymethyl, 2,2-dihydroxyethyl, dihydroxypropyl and the like. Examples of sulfoalkyl groups include sulfo-C₁₋₄ alkyl groups such as sulfomethyl, 2-sulfoethyl, sulfobutyl, and the like. Examples of carboxyalkyl groups include carboxy-C₁₋₄ alkyl groups such as carboxymethyl, 2-carboxyethyl, and the like. Examples of sugar moieties include glucosyl, maltosyl, panosyl, and the like.

Compounds preferable for use as the cyclodextrin compound in the present invention include α-cyclodextrins and derivatives thereof represented by formula (1) wherein n is 6; β-cyclodextrins and derivatives thereof represented by formula (1) wherein n is 7; γ-cyclodextrins and derivatives thereof represented by formula (1) wherein n is 8; and δ-cyclodextrins and derivatives thereof represented by formula (1) wherein n is 9. Examples of such preferable cyclodextrin derivatives include alkyl derivatives of cyclodextrin, hydroxyalkyl derivatives of cyclodextrin, sulfoalkyl ether derivatives of cyclodextrin, and sugar-substituted derivatives of cyclodextrin. Specific examples of the alkyl derivatives include dimethyl-α-cyclodextrin, dimethyl-β-cyclodextrin, dimethyl-γ-cyclodextrin, and the like. Examples of the hydroxyalkyl derivatives include 2-hydroxypropyl-α-cyclodextrin, 2-hydroxypropyl-β-cyclodextrin, 2-hydroxypropyl-γ-cyclodextrin, and the like. Examples of the sulfoalkylether derivatives include sulfobutylether-α-cyclodextrin, sulfobutylether-β-cyclodextrin, sulfobutylether-γ-cyclodextrin, and the like. Examples of the sugar-substituted cyclodextrin derivatives include glucosyl-α-cyclodextrin, glucosyl-β-cyclodextrin, glucosyl-γ-cyclodextrin, maltosyl-α-cyclodextrin, maltosyl-β-cyclodextrin, maltosyl-γ-cyclodextrin, and the like. Among these, 2-hydroxypropyl-β-cyclodextrin and sulfobutylether-β-cyclodextrin are particularly preferable.

Examples of pharmaceutically acceptable salts of cyclodextrins and cyclodextrin derivatives include sodium salts, potassium salts, magnesium salts, and the like.

The amount of cyclodextrin compound incorporated into the fat emulsion of the invention is usually selected from the range of about 0.02 to about 1 w/v%, preferably about 0.05 to about 0.5 w/v%, and further preferably about 0.05 to 0.2 w/v%. A propofol-containing fat emulsion that achieves the intended effects of the invention without reducing the safety of the resulting fat emulsion can be obtained by incorporating the cyclodextrin compound in the above-mentioned range.

### Other additives

Although not necessary, if desired, various additives known for addition to fat emulsions may also be incorporated in appropriate amounts in the propofol-containing fat emulsion of the invention or the emulsion used to prepare the emulsion of the invention. Examples of such additives include antioxidants, antibacterial agents, pH adjusting agents, isotonizing agents, and the like. Specific examples of antioxidants include sodium metabisulfite (which also acts as an antibacterial agent), sodium sulfite, sodium bisulfite, potassium metabisulfite, potassium sulfite, sodium thiosulfate, and the like. Examples of antibacterial agents include sodium caprylate, methyl benzoate, sodium metabisulfite (which also acts as an antioxidant), sodium edetate, and the like. Examples of pH adjusting agents include hydrochloric acid, acetic acid, lactic acid, malic acid, citric acid, sodium hydroxide, and the like. Examples of isotonizing agents include glycerol; saccharides such as glucose, fructose, maltose, and the like; and sugar alcohols such as sorbitol, xylitol, and the like. Among these, oil-soluble additives can be mixed beforehand with an oily component that will form the emulsion, and water-soluble additives can be mixed with water for injection or admixed to the aqueous phase of the obtained emulsified liquid. The amounts of such additives are known to persons skilled in the art and are not substantially different from conventionally-employed amounts.

Further, if desired, stabilizers for improving emulsion stability can be incorporated into the propofol-containing fat emulsion of the invention, or the emulsion used to prepare the fat emulsion. Examples of such stabilizers include commonly used surfactants, and the like. Examples of such surfactants include the following substances (a) to (c) whose effects of stabilizing this type of fat emulsion were found by the present inventors (see International Publication No. WO 2004/052354 pamphlet), the disclosure of which is incorporated in this specification by reference.
(a) At least one phospholipid selected from the group consisting of phosphatidylglycerols, phosphatidic acids, phosphatidylinositols and phosphatidylserines wherein each fatty acid esterified to a glycerol moiety is a C₁₀₋₂₂, and preferably C₁₂₋₁₈, linear or branched, saturated or unsaturated fatty acid;
(b) at least one phospholipid derivative selected from the group consisting of phosphatidylethanolamines modified with polyalkyleneglycol, wherein each fatty acid esterified to a glycerol moiety is a C₁₀₋₂₂, and preferably C₁₄₋₁₈, linear or branched, saturated or unsaturated fatty acid; and
(c) at least one fatty acid selected from the group consisting of C₁₀₋₂₂, and preferably C₁₀₋₂₀, linear or branched, saturated or unsaturated fatty acids.

### Fat emulsion of the invention

The fat emulsion of the invention is prepared by mixing and emulsifying the above-mentioned propofol, oily component, emulsifier and cyclodextrin compound in specific amounts, optionally with additives.

Examples of particularly preferable combinations of components include a combination of soybean oil as the oily component, egg yolk lecithin as the emulsifier, and at least one member selected from the group consisting of 2-hydroxypropyl-β-cyclodextrin and sulfobutylether-β-cyclodextrin as the cyclodextrin compound.

Table 1 below shows preferable proportions and particularly preferable proportions (final concentrations) of the components.

**[Table 1]**

| Component | Preferable proportion | Particularly preferable proportion |
|---|---|---|
| Propofol | 0.1 to 5 w/v% | 0.5 to 3 w/v% |
| Oily component | 2 to 20 w/v% | 3 to 10 w/v% |
| Emulsifier | 0.4 to 10 w/v% | 0.5 to 7 w/v% |
| Cyclodextrin compound | 0.02 to 1 w/v% | 0.05 to 0.2 w/v% |
| Water for injection | q.s. | |

Methods of mixing and emulsifying the components are not particularly limited as long as an emulsion can be obtained. The mixing and emulsifying procedures can be carried out according to known methods. For example, the intended fat emulsion of the invention can be obtained by a process comprising emulsifying a mixture comprising propofol, an oily component and an emulsifier in water, and then adding a cyclodextrin compound to the obtained emulsion.

The intended fat emulsion of the invention can also be obtained by a process comprising adding a mixture comprising propofol, an oily component and an emulsifier to an aqueous solution of a cyclodextrin compound, and then emulsifying the obtained mixture.

Further, the intended fat emulsion of the invention can also be obtained by preparing a fat emulsion comprising an oily component and an emulsifier, adding propofol and a cyclodextrin compound to the obtained fat emulsion, and then emulsifying the resulting mixture.

Means for preparing an emulsified liquid usable in the above-mentioned processes (emulsification methods) are well known in this field. For example, a means comprising stirring at 5000 rpm or more for at least 5 minutes using, for example, a homomixer such as a T.K. homomixer manufactured by Tokushu Kika Kogyo Co., Ltd. to produce a crude emulsion, and a means using a high-pressure homogenizer, an ultrasonic homogenizer, or the like to refine the crude emulsion can be used. When using a high-pressure homogenizer, the crude emulsion can be usually refined by passing through the homogenizer about 2 to 50 times, and preferably about 5 to 20 times, at a pressure of about 200 kg/cm² or more. The mixing and emulsifying processes can be carried out at room temperature or with slight heating (usually about 55°C to 80°C).

The thus-obtained fat emulsion of the invention can be adjusted to a desired pH value, if necessary, and then filtrated and sterilized according to known methods to give a final product.

The pH of the propofol-containing fat emulsion of the invention is usually adjusted to about 5.0 to about 9.0, and preferably about 6.0 to about 8.0.

Filtration can be carried out using a known membrane filter. Sterilization can be performed, for example, using high-pressure steam sterilization, hot water immersion sterilization, shower sterilization, and the like. Examples of preferable sterilization procedures include high-pressure steam sterilization using an autoclave (for example, 121°C for 12 minutes).

The present invention further provides a method of reducing vascular pain caused by intravenous or infusion administration of a propofol-containing fat emulsion, the method comprising incorporating 0.02 to 1 w/v% of a cyclodextrin compound into the fat emulsion.

The method can be carried out in the same manner as the process for preparing the fat emulsion of the invention, i.e., the process of mixing and emulsifying the components.

The present invention further provides the use of a cyclodextrin compound for preparing the propofol-containing fat emulsion of the invention.

### EFFECTS OF THE INVENTION

In the fat emulsion of the present invention, the combined use of the specific amounts of propofol, oil component, emulsifier and cyclodextrin compound, and in particular the use of the specific amount of cyclodextrin compound, achieves the remarkable effect of reducing the vascular pain caused by the administration of a propofol-containing fat emulsion.

Since the cyclodextrin compound is used in such a small amount that does not cause problems with toxicity, side effects (hemolysis), etc., the fat emulsion of the present invention is highly safe.

Further, the fat emulsion of the present invention has excellent emulsion stability, and in particular excellent emulsion stability against temperature change. Specifically, even when the fat emulsion of the present invention is subjected to a heat sterilization process such as high-pressure steam sterilization, it retains its emulsion stability without being deteriorated by the sterilization process. The fat particles in the fat emulsion of the invention have a mean diameter of as small as not more than about 0.3 µm, which is substantially the same before and after sterilization. Moreover, such excellent emulsion stability is maintained over long-term storage (e.g., at 40°C for 6 months). The fat emulsion of the present invention not only retains its excellent emulsion stability for a long time, but also has an excellent feature in that the activity of propofol, i.e., the active ingredient, is not substantially decreased during heat sterilization and subsequent long term storage. The present inventors confirmed that the propofol activity is not substantially decreased when the fat emulsion of the present invention is stored at 40°C for 6 months.

### BRIEF DESCRIPTION OF THE DRAWINGS

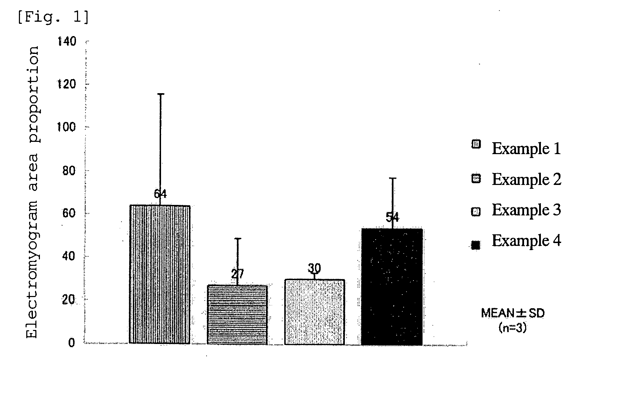

[Fig. 1] A graph showing the degree of vascular pain reduction when administering the propofol-containing fat emulsion of the present invention.

### BEST MODE FOR CARRYING OUT THE INVENTION

The following Examples describe the present invention in further detail.

### Examples 1 to 4

(1) Fat emulsions of the present invention (each 100 ml in total volume) consisting of the components shown in Table 2 were prepared as described below.

**[Table 2]**

| Component | Amount per 100 ml | | | | |
|---|---|---|---|---|---|
| | Control (1% Diprivan™ injection) | Ex. 1 | Ex. 2 | Ex. 3 | Ex. 4 |
| HP-β-CyD | - | 0.02 g | 0.05 g | 0.1 g | 0.2 g |
| Propofol | 1 g | 1 g | 1 g | 1 g | 1 g |
| Soybean oil | 10 g | 10 g | 10 g | 10 g | 10 g |
| Egg yolk lecithin | 1.2 g | 1.2 g | 1.2 g | 1.2 g | 1.2 g |
| Glycerol | 2.21 g | 2.21 g | 2.21 g | 2.21 g | 2.21 g |
| Water for Injection | q.s. | q.s. | q.s. | q.s. | q.s. |

The components shown in Tables 2 are the following products.
Propofol: product of ALBEMARLE
Soybean oil: purified soybean oil manufactured by Nisshin Oil Mills, Ltd.
Egg yolk lecithin: purified egg yolk lecithin manufactured by Q.P. Corp.
HP-β-CyD (2-hydroxypropyl-β-cyclodextrin): "Cavitron™ 82003" manufactured by Cargill

According to the proportions shown in Table 2, the propofol and soybean oil were mixed, and then the egg yolk lecithin was added. Thereafter, the glycerol dissolved in water for injection so that the final concentration would become 2.21 w/v% was further added, and the resulting mixture was crudely emulsified in a POLYTRON™ homogenizer (manufactured by
KINEMATICA) at 25000 rpm for 10 minutes under a nitrogen stream with heating. The HP-β-CyD was then added to the resulting crude emulsified liquid.

Subsequently, using a high-pressure homogenizer (manufactured by APV), the crude emulsified liquid was converted into a fine emulsion with a mean particle diameter of not more than 0.3 µm, under a nitrogen stream at an emulsification temperature of 40°C to 80°C and an emulsification pressure of 550 kg/cm².

After adjusting the emulsion to a predetermined pH (pH 5 to 8) with hydrochloric acid or sodium hydroxide, 10 ml of the emulsion was placed in a 10 ml glass vial, sealed and subjected to high-pressure steam sterilization. Fat emulsion samples were thus obtained.

The fat emulsions thus obtained had excellent emulsion stability at pH 5 to 8, such that no crystalline precipitation was observed even after being subjected to high-pressure steam sterilization. The emulsified particles had small uniform diameters of about 200 nm.

Using the obtained fat emulsion samples, the following vascular pain assessment test was performed. In this test, a commercially available 1% Diprivan™ injection (manufactured by AstraZeneca and consisting of the components as shown in Table 2) was used.

### Test Example 1 (vascular pain assessment test)

One of the fat emulsion samples was administered to the femoral artery of a rat, and the degree of vascular pain was assessed by electromyography of a site adjacent to the artery to which the sample had been administered. Vascular pain assessment by this electromyographic method is described, for example, in the following reference: R. Ando, A. Yonezawa, C. Watanabe and S. Kawamura, "An assessment of vascular pain using the flexor reflex in anesthetized rats", Methods Find. Exp. Clin. Pharmacol., 2004 Mar; 26(2): 109-15.

The test was performed as follows. Male 7- to 9-week-old SD rats were divided into 4 groups, and each group (n=3) was subjected to urethane anesthesia. After shaving the right hind leg operative field and the electrode insertion site, the rats were fixed in the supine position. For administration of the fat emulsion samples, a polyethylene catheter was placed in the right superficial caudal epigastric artery, and the rats were fixed in the prone position in a Ballman cage. A coaxial needle electrode and indifferent electrode for electromyography were placed on the right hind leg, and connected to a bioelectric amplifier (AB-621G, Nihon Kohden Corp.).

Electromyographic recordings were started before administration of the fat emulsion samples. When at least one hour had passed after the operation and the electromyographic waveforms had stabilized, 0.05 ml of 1% Diprivan™ injection was administered through the polyethylene catheter. After the administration, electromyography was performed to calculate the area under the peak in the electromyogram (the calculated value was used as a reference).

One hour after the administration of 1% Diprivan™ injection, the fat emulsion samples of Examples 1 to 4 (each 0.05 ml) adjusted to pH 8 were administered to the four rat groups, respectively, and after the administration, the areas under the peaks of the electromyographic waves were calculated.

The value obtained from each rat in each group was compared with the reference previously obtained from the same rat to determine its percentage (hereinafter referred to as "electromyogram area proportion", expressed in %), which was used as an index of vascular pain. The index obtained by the above test is free from individual differences in sensitivity to pain. When this index is less than 100%, it indicates that the vascular pain is reduced as compared with administration of 1% Diprivan™ injection. Further, the smaller the index (electromyogram area proportion), the greater the vascular pain reducing effect.

Fig. 1 shows the electromyogram area proportions (%) obtained from the fat emulsion sample administration groups (Groups 1 to 4), as the mean±SD of the rats in each group.

Fig. 1 reveals that the areas under the peaks in the electromyograms after the administration of the fat emulsion samples (prepared in Examples 1 to 4) are remarkably reduced as compared with the area before the administration of the samples (after administration of 1% Diprivan™ injection). This demonstrates that the fat emulsion of the present invention can greatly reduce the vascular pain caused by administration of a propofol-containing fat emulsion.

### INDUSTRIAL APPLICABILITY

The propofol-containing fat emulsion of the present invention has excellent safety while retaining emulsion stability, can prevent or reduce the pain caused by the administration of a propofol-containing fat emulsion, and is useful as a medicine such as general anesthetic, sedative or the like.

## Claims

1. A propofol-containing fat emulsion capable of being administered with reduced vascular pain during intravenous or infusion administration, the emulsion comprising 0.1 to 5 w/v% of propofol, 2 to 20 w/v% of an oily component, 0.4 to 10 w/v% of an emulsifier and 0.02 to 1 w/v% of at least one compound selected from the group consisting of cyclodextrins, cyclodextrin derivatives and pharmacologically acceptable salts thereof.

2. The fat emulsion according to claim 1 wherein the oily component is at least one compound selected from the group consisting of natural triglycerides and synthetic triglycerides, and the emulsifier is at least one member selected from the group consisting of natural phospholipids and synthetic phospholipids.

3. The fat emulsion according to claim 2 wherein the oily component is soybean oil, and the emulsifier is egg yolk lecithin.

4. The fat emulsion according to claim 1 wherein the at least one compound selected from the group consisting of cyclodextrins, cyclodextrin derivatives and pharmacologically acceptable salts thereof is at least one member selected from the group consisting of 2-hydroxypropyl-β-cyclodextrin and sulfobutylether-β-cyclodextrin.

5. The fat emulsion according to claim 1 wherein the oily component is soybean oil, the emulsifier is egg yolk lecithin, and at least one compound selected from the group consisting of cyclodextrins, cyclodextrin derivatives and pharmacologically acceptable salts thereof is at least one member selected from the group consisting of 2-hydroxypropyl-β-cyclodextrin and sulfobutylether-β-cyclodextrin.

6. The fat emulsion according to claim 1 comprising 0.5 to 3 w/v% of propofol, 3 to 10 w/v% of the oily component, 0.5 to 7 w/v% of the emulsifier, and 0.05 to 0.5 w/v% of at least one compound selected from the group consisting of cyclodextrins, cyclodextrin derivatives and pharmacologically acceptable salts thereof.

7. The fat emulsion according to claim 1 comprising 0.5 to 3 w/v% of propofol, 3 to 10 w/v% of the oily component, 0.5 to 7 w/v% of the emulsifier, and 0.05 to 0.2 w/v% of at least one compound selected from the group consisting of cyclodextrins, cyclodextrin derivatives and pharmacologically acceptable salts thereof.

8. A process of preparing the fat emulsion of claim 1 comprising emulsifying a mixture comprising propofol, the oily component and the emulsifier in water, and then adding to the obtained emulsion at least one compound selected from the group consisting of cyclodextrins, cyclodextrin derivatives and pharmacologically acceptable salts thereof.

9. A process of preparing the fat emulsion of claim 1 comprising adding a mixture comprising propofol, the oily component and the emulsifier to an aqueous solution of at least one compound selected from the group consisting of cyclodextrins, cyclodextrin derivatives and pharmacologically acceptable salts thereof, and then emulsifying the obtained mixture.

10. A method of reducing vascular pain caused by intravenous or infusion administration of a fat emulsion comprising 0.1 to 5 w/v% of propofol, 2 to 20 w/v% of an oily component and 0.4 to 10 w/v% of an emulsifier, the method comprising the step of incorporating into the fat emulsion 0.02 to 1 w/v% of at least one compound selected from the group consisting of cyclodextrins, cyclodextrin derivatives and pharmacologically acceptable salts thereof.

11. Use of at least one compound selected from the group consisting of cyclodextrins, cyclodextrin derivatives and pharmacologically acceptable salts thereof for preparing a propofol-containing fat emulsion capable of being administered with reduced vascular pain during intravenous or infusion administration, the emulsion comprising 0.1 to 5 w/v% of propofol, 2 to 20 w/v% of an oily component, 0.4 to 10 w/v% of an emulsifier and 0.02 to 1 w/v% of at least one compound selected from the group consisting of cyclodextrins, cyclodextrin derivatives and pharmacologically acceptable salts thereof.
